Europäisches Patentamt

(19) European Patent Office

Office européen des brevets

(11) Publication number : **0 382 720 B1**

# (12) EUROPEAN PATENT SPECIFICATION

(45) Date of publication of patent specification :
09.09.92 Bulletin 92/37

(21) Application number : **88903655.4**

(22) Date of filing : **29.03.88**

(86) International application number :
**PCT/US88/00999**

(87) International publication number :
**WO 88/07681 06.10.88 Gazette 88/22**

(51) Int. Cl.⁵ : **G01N 33/53,** G01N 33/92,
G01N 33/00, C12N 15/00

㉞ DIAGNOSING OBESITY CAUSED BY A GENETIC ABNORMALITY AND METHOD OF
THERAPEUTICALLY TREATING GENETICALLY CAUSED OBESITY.

(30) Priority : **02.04.87 US 34203**

(43) Date of publication of application :
**22.08.90 Bulletin 90/34**

(45) Publication of the grant of the patent :
**09.09.92 Bulletin 92/37**

(84) Designated Contracting States :
**AT BE CH DE FR GB IT LI LU NL SE**

(56) References cited :
**CLINICAL RESEARCH, vol. 35, no. 3, March
1987, US; J.S.FLIER et al., p. 622a
SCIENCE, vol. 237, 24 July 1987, Washington,
DC (US); COOK et al., pp. 402-405
SCIENCE, vol. 237, 24 July 1987, Washington
DC (US); FLIER et al., pp. 405-408
PROCEEDINGS OF THE NATL. ACADEMY OF
SCIENCES USA, vol. 82, October1985,
Washington, DC (US); COOK et al., pp.
6480-6484**

(56) References cited :
**PROCEEDINGS OF THE NATL. ACADEMY OF
SCIENCES USA, vol. 83, June 1986, Washing-
ton, DC (US); HUNT et al., pp. 3786-3790
NUCLEIC ACIDS RESEARCH, vol. 14, no. 22,
1986, Oxford, England (GB); MIN et al., pp.
8879-8892**

(73) Proprietor : **THE BETH ISRAEL HOSPITAL
ASSOCIATION
330 Brookline Avenue
Boston, MA 02215 (US)**
Proprietor : **DANA-FARBER CANCER
INSTITUTE
44 Binney Street
Boston, MA 02115 (US)**

(72) Inventor : **FLIER, Jeffrey, S.
14 Sylvan Avenue
West Newton, MA 02165 (US)**
Inventor : **SPIEGELMAN, Bruce, M.
41 Oakmont Road
Newton, MA 02159 (US)**

(74) Representative : **Bassett, Richard Simon et al
ERIC POTTER & CLARKSON St. Mary's Court
St. Mary's Gate
Nottingham NG1 1LE (GB)**

## Description

BACKGROUND OF THE INVENTION

This invention relates to a method to distinguish between individuals who have normal metabolisms and gain weight due to overeating and those individuals who have a propensity to gain weight due to one or more genetic or metabolic abnormalities.

Obesity is a condition which results when a living being has an excessive adipose (fat) cell mass. Thus, a patient becomes obese when there is a disorder in his or her systemic energy balance that leads to an increased adipose cell mass.

A normal metabolic system responds to overeating with increased sympathetic nervous system activity and, as a consequence, increased thermogenesis of heat production. Such heat production in mammals appears to serve as a physiologic defense against excessive weight gain. When individuals eat beyond their metabolic limits, however, no amount of sympathetic nervous activity will stop them from gaining weight and becoming obese. A subset of obese individuals, however, have genetically determined metabolic defects such as a deficiency in heat generation which increases metabolic efficiency and weight gain. Stated another way, many individuals have healthy or normal weight levels because their metabolic systems are capable of balancing their caloric intake. Other individuals are obese because their caloric intake is excessively high and their adaptive increase in metabolic rate is not sufficient to counteract the excessive caloric intake. Such individuals have the ability to lose weight by reducing the amount of food they eat, by increasing the amount of exercise or both. However, another group of individuals exists whose tendency to gain weight is promoted by a metabolic, neuroendocrine or genetic defect. That is, they appear to ingest food with caloric content equivalent to that of many normal weight individuals, yet they continue to gain weight in an unacceptable fashion. This latter category are said to be genetically or metabolically obese.

Recognizing which patients have these genetic or metabolic defects is vital in order to properly categorize and advise these patients, and ultimately impact a physician's ability to safely administer treatment for weight control. prescribing a low calorie diet to a patient suffering from genetically induced obesity may not yield satisfactory results and may, in fact, have a deleterious effect on the patient's health. It is, of course, desirable to know if weight gain is due to genetic problems or simply overeating. If the weight gain is due to simply overeating, a proper diet can be prescribed to achieve weight loss. On the other hand, if the weight gain is due to genetically or metabolically induced obesity, then some other form of treatment is in order. Despite numerous investigations prior to the present invention, which span such varied disciplines as psychiatry, neuroendocrinology, genetics and metabolism, few clear pathogenetic insights have emerged at the molecular level. The present invention diagnoses and treats genetically or metabolically caused obesity at the molecular level.

Obesity is considered a disorder of systemic energy balance. However, investigators have believed that molecular defects operate at a variety of levels in the obese patient, involving components of the systems responsible for regulation of food intake and energy expenditure. The role of the adipose cell itself, as either an active participant in the systemic dysfunction of obesity or a passive responder to external signals, has been studied. However, its effects are unknown. Although evidence has not been compelling, theories invoking a putative fat cell-derived signal that might exert systemic effects on appetite control or energy expenditure (i.e. the adipostat) have been proposed. The possibility that reduced expression of the membrane Na,K-ATPase might be a marker for characterizing obese states or distinguishing different classes of obese patients has also been proposed, but neither this marker nor any other marker has been able to clearly define pathogenetically distinct subgroups of obese patients.

In a separate field of interest, it has been found that many new proteins are expressed when preadipocyte cell lines differentiate into adipocytes in culture. A number of cDNA clones corresponding to the mRNAs that encode these proteins have been isolated. Although some of these have been shown to be key enzymes of lipid metabolism, others represent novel gene products. One particular gene product is named adipsin. Adipsin is a member of the serine protease family which is expressed abundantly in adipose cells. The protein adipsin is secreted by cells that express it and is present in the peripheral circulation.

SUMMARY OF THE INVENTION

In accordance with the present invention, it has been discovered that the expression of the putative serine protease called adipsin distinguishes those forms of obesity which arise from certain genetic or metabolic-defects from those which result in pure overeating. The reduced expression of adipsin clearly defines pathogenetically distinct subgroups of obese patients. Furthermore, these observations demonstrate that the local and systemic adipsin level is a factor in the pathophysiology of altered energy balance in obesity and is an essential aspect of the proper treatment of genetically or metabolically obese individuals.

Accordingly, the present invention is a blood test and a tissue biopsy test that will give an indication of the cause of a patient's obesity. It has been found that a low level of adipsin protein in the peripheral blood and a low level of adipsin protein and mRNA in adipose tissue are indications of a genetic defect that will cause obesity. In contrast, no diminution in adipsin level is observed in a model of obesity based on pure overeating by normal individuals. Quantification of the adipsin protease is possible by assaying a patient's blood or tissue sample with a labelled antibody which recognizes the human protein adipsin. Another method of quantification is assaying a patient's tissue sample with a cDNA or genomic DNA probe. Detection of adipsin deficiency enables the safe treatment of patients with genetic or metabolic defects. With this knowledge, physicians may provide effective treatment for these patients by prescribing a dosage of the adipsin protein itself.

It is therefore an object of the present invention to provide a method to distinguish between individuals who have normal metabolism and gain weight due to overeating and those individuals who have a propensity to gain weight due to a genetic or metabolic abnormality.

It is another object of the present invention to provide a method to detect the level of adipsin in a patient.

It is another object of the present invention to provide a method to distinguish between individuals who have normal metabolism and gain weight due to overeating and those individuals who have a propensity to gain weight due to a genetic or metabolic abnormality by providing an assay kit for measuring the amount of adipsin in the patient's blood stream or adipose tissue.

It is yet another object of the present invention to provide a method to distinguish between individuals who have normal metabolism and gain weight due to overeating and those individuals who have a propensity to gain weight due to a genetic or metabolic abnormality by providing an assay kit for measuring the amount of adipsin mRNA in the patient's adipocytes.

## BRIEF DESCRIPTION OF THE DRAWING

FIG. I illustrates the relative amounts of mRNA for glycerophosphate dehydrogenase (GPD), adipsin (ADN), aP2 and actin (ACT) in 10 μg samples of total cellular RNA that was extracted from epidydimal fat pads, electrophoresed on a I.2% formaldehyde agarose gel, transferred to a nylon filter and probed with the indicated cDNAs. Three sets of samples were taken from 200 gram male Sprague Dawley (SD) rats which were allowed ad libitum access to standard lab chow (fed), deprived of chow for 3 days (fasted) or refed with chow for 5 days after a 3-day fast (refed).

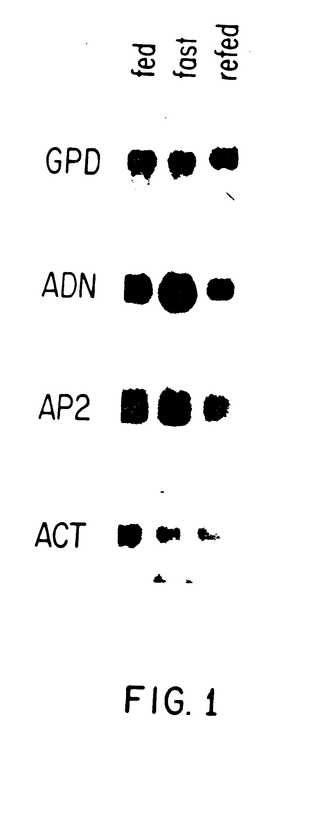

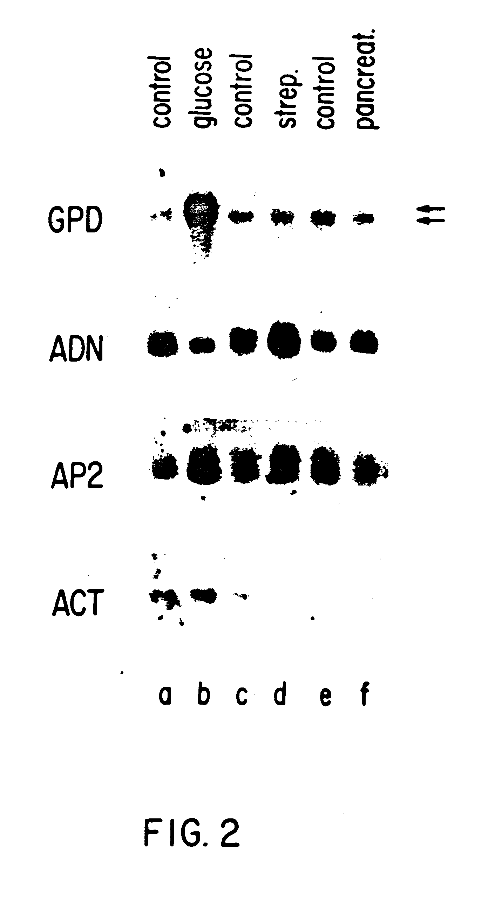

FIG. 2 illustrates the relative amounts of the above mentioned mRNAs, in epidydimal fat pad RNA similarly prepared, in three sets of rats. In the first (glucose), SD rats were infused with dextrose (lane b) or saline (lane a) for 48 hours. In the second model (strep), SD rats were injected with 40mg/kg of streptozotocin (lane d) and studied 3 weeks later. The control rat (lane c) was age-matched untreated. In the third model, SD rats were subjected to 90% surgical pancreatectomy (lane f) or sham operated (lane e).

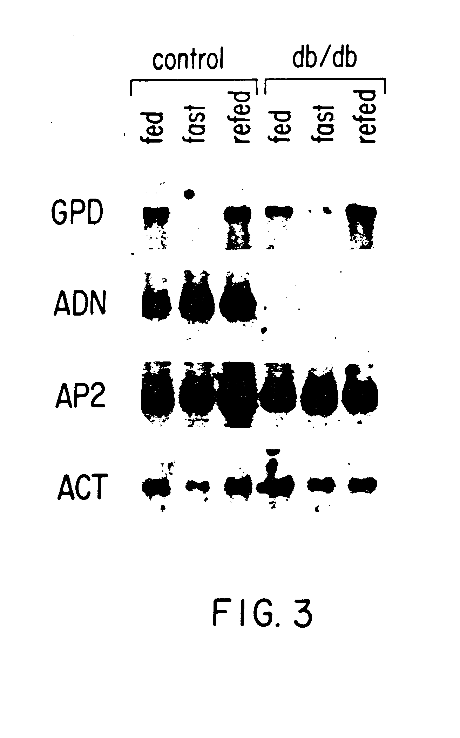

FIG. 3 illustrates the relative amounts of the above mentioned mRNAs, in 5μg samples of epidydimal fat pad RNA similarly prepared, in three sets of rat studies. Male db/db mice of 8-I2 weeks of age and control age-matched congenic C57BL/K5J mice were allowed ad libitum access to chow (fed), deprived of chow for 3 days (fast) and refed for 3 days with chow after a 3-day fast (refed).

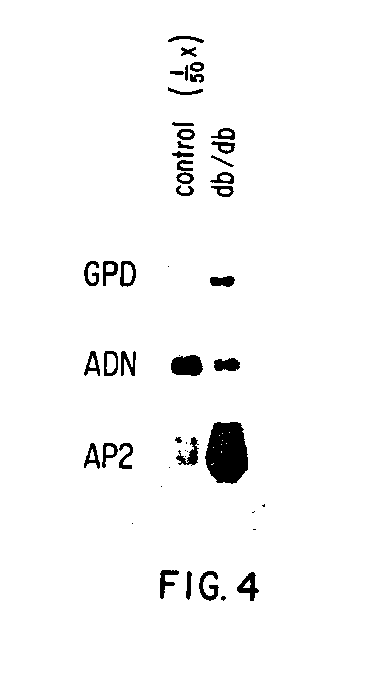

FIG. 4 illustrates the comparison of signal intensities for specific mRNAs in total epidydimal fat pad RNA from control and db/db mice with ad lib feeding. For detection of ADN RNA, the db/db RNA was loaded in 50-fold excess relative to that in the control lane.

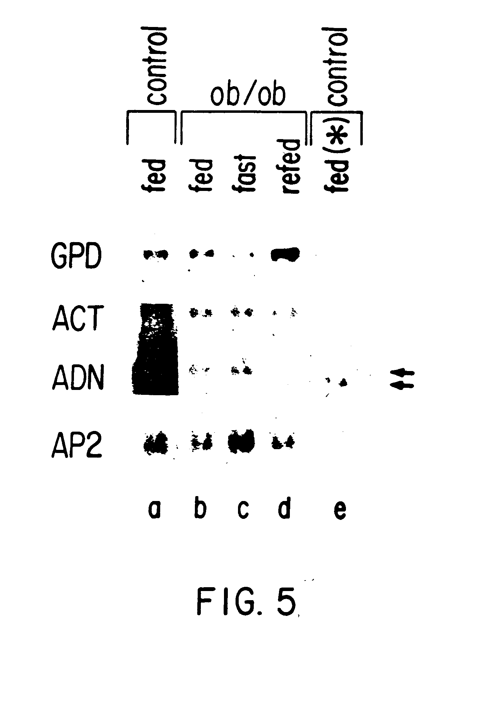

FIG. 5 illustrates the relative amounts of the above mentioned mRNAs, in similarly prepared epidydimal fat pad RNA, in three sets of rat studies. Male ob/ob mice of 8-I2 weeks of age and control age-matched congenic C57BL/6J were studied while on ad lib chow (fed) (lane a,b,e), after 3 days deprivation of chow (fast) (lane c) and after 3 days of refeeding following a 3-day fast (refed) (lane d). Lane e is a 50-fold shorter exposure of lane a, to permit a more direct comparison of mRNA size in ob/ob and control. Arrows point to adipsin species in ob/ob and control mice.

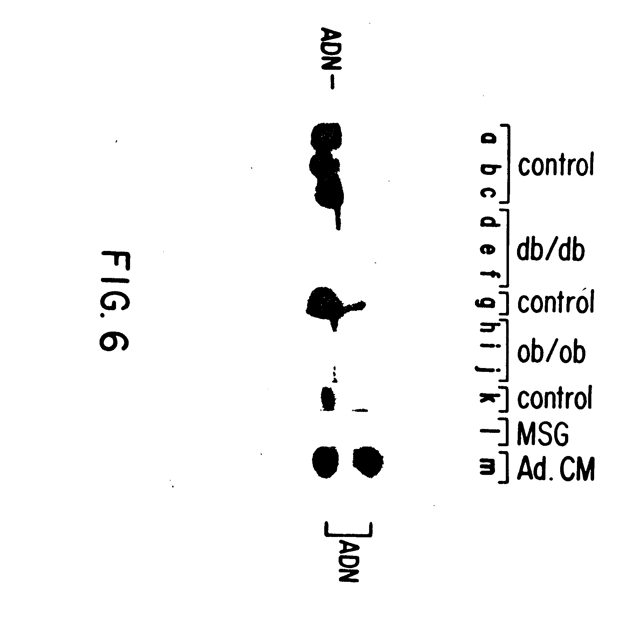

FIG. 6 illustrates the results of Western blotting of adipose tissue extracts with adipsin antisera. Lane a-c (control) were from fed (a), 3-day fasted (b), and refed (c) controls congenic to db/db animals, and d-f were fed (d), fasted (e), and refed (f) db/db mice. Lane g is ad lib fed control mouse congenic to the ob/ob, and h-j (ob/ob) are fed (h), fasted (i) and refed (j) ob/ob mice. Lane k is an ad lib fed control age-matched with the mouse in lane I. Lane I (MSG) is from a mouse treated neonatally width subcutaneous injection of monosodium glutamate (3mg/g body weight) and studied I2 weeks later. Lane m is conditioned medium from cultured 3T3 F442A adipocytes.

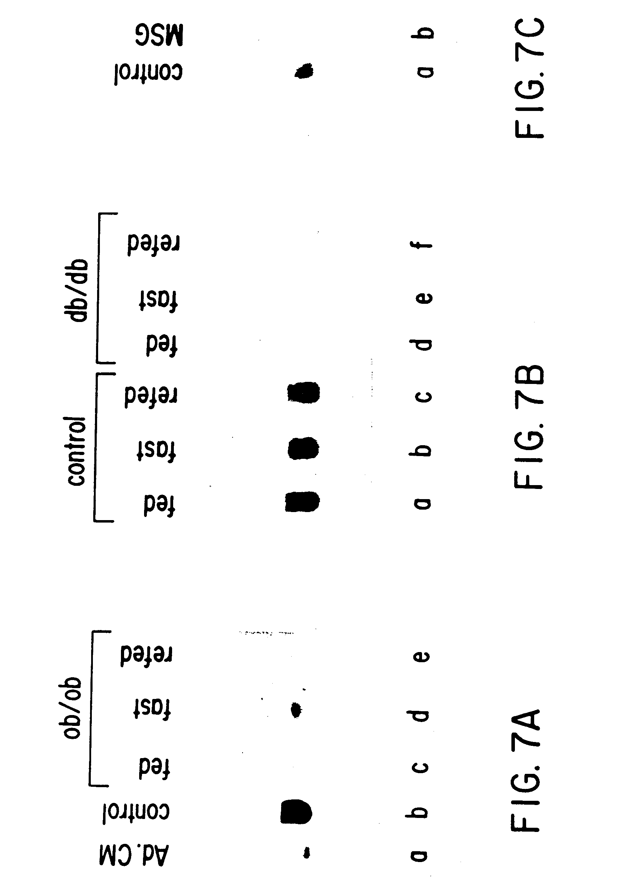

FIG. 7 illustrates an immunoblot of sera from normal and obese mice. 5g of each serum was electrophoresed, immunoblotted and probed with anti adipsin.

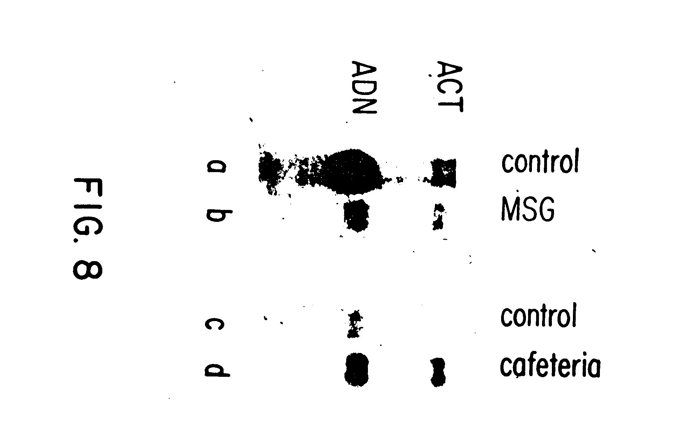

FIG. 8 illustrates the Northern blotting results of RNA from adipose tissue of mice with MSG-induced obesity or cafeteria-feeding.

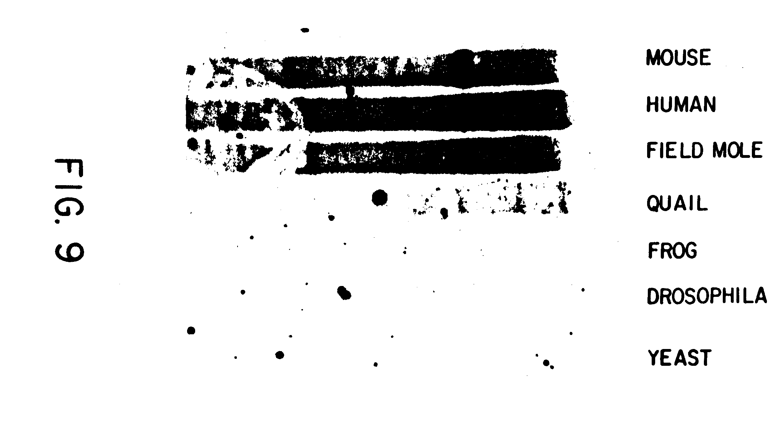

FIG. 9 illustrates the Southern blotting results indicating that the human adipsin gene can be detected with the mouse adipsin cDNA probe.

## DESCRIPTION OF THE PREFERRED EMBODIMENTS

At the outset, the invention is described in its broadest overall aspects with a more detailed description following. The broadest aspects of the invention involve assaying an obese patient's blood sample for the presence of the protein adipsin or assaying an obese patient's adipose tissue sample for the presence of the adipsin protein or mRNA and administering proper treatment. In those instances where the test shows normal levels of adipsin, proper treatment for an obese patient will typically be a regimen including proper diet and exercise. On the other hand, if the test indicates that the patient's obesity is due in part to a deficiency in adipsin levels, then the physician will administer therapeutically effective amounts of adipsin to the patient.

The present invention builds on the prior discovery that adipsin is a novel member of the serine protease gene family. R.S. Cook, D.L. Groves, H.Y. Min, B.M. Spiegelman, 82 Proc. Nat. Acad. Sci. USA 6480 (l985). It was discovered through cloning and sequencing of its cDNA. Adipsin is expressed abundantly and relatively specifically in adipose cells. K.M. Zusalak, H. Green, 5 J. Mol. Cell. Biol. 4l9 (l985). Prior to the present invention, however, the physiologic function of adipsin was not known. The fact that adipsin is a fat-derived protein raised the possiblity that it may have a systemic function, perhaps a role in the regulation of energy balance. In accordance with the present invention, however, adipsin levels correlate with, and may regulate, the increase in adipose mass. That is, an adipsin deficiency in a patient permits an excessive enlargement of the adipose mass resulting in an accumulation of fat and the development of obesity. In accordance with the present invention, the role of adipsin was assessed. To begin an assessment, two experimental approaches were taken. The first involved examining the effect of several nutritional and metabolic perturbations upon adipsin gene expression. The second involved assessment of adipsin gene expression in several distinct models of rodent obesity.

The regulation of several adipocyte genes during various catabolic or anabolic perturbations in rat adipose cells and tissue was examined. The results of these investigations are shown in FIGS. l and 2. Rats were fasted for 3 days, or fasted for 3 days followed by 5 days of refeeding. Epidydimal adipocyte mRNA was isolated from these rats and analyzed by Northern blotting. Isolated rat adipocytes were prepared using the collagenase technique and RNA was extracted with guanidinum isothiocyanate and CsCl centrifugation. Total cellular RNA (l0g) was electrophoresed on a l.2% formaldehyde agarose gel, transferred to a nylon filter and probed with cDNAs for glycerophosphate dehydrogenase (GPD), a key lipogenic enzyme, adipsin (ADN) and aP2, a putative lipid binding protein. Radiolabelling was by the random priming method. Hybridization of the mRNA with the cDNAs and washing were performed as described by Spiegelman et al. B.M. Spiegelman, M. Frank, H. Green, 258 J. Biol. Chem. l0083 (l983). Autoradiograms were-scanned by laser densitometry and all values were normalized to the signal obtained for actin mRNA. This normalization caused no more than a 30% change from that when the results were based per unit of total RNA.

FIG. l illustrates that starvation caused little change in GPD mRNA and only a small increase (40%) in aP2 mRNA level. In contrast, adipsin mRNA was increased approximately 5-fold by fasting. Refeeding led to a drop in this message back to or below the level of the ad libidum fed control. Actin mRNA, used as a control, undergoes a small decrease (25%) during this protocol and the quantitative changes reported in aP2 and adipsin mRNA are corrected for this general loss of mRNA due to fasting. Identical results were obtained when mRNA was prepared from whole epidydimal adipose tissue instead of isolated fat cells (not shown).

In the strep model in FIG. 2, rats were injected with 40 mg of streptozotocin per kilogram of body weight (lane d) and studied 3 weeks later. The control rat (lane c) was age-matched and untreated. The blood glucose level of the strep animal was 450 mg/dl. Streptozotocin-induced diabetes is also characterized by hyperglycemia, but, unlike direct glucose infusion, these animals are in a catabolic state because of the deficiency in insulin. In this case, there is no increase in GPD mRNA as shown in FIG. 2, indicating that hyperglycemia alone is not sufficient to bring about the induction of GPD. Adipsin mRNA is induced 4-fold, again suggesting that the expression of this gene is linked directly or indirectly to some aspect of the catabolic state. aP2 mRNA undergoes little change.

In the third model (pancreatectomy), rats were subjected to 90% surgical pancreatectomy (lane f) or sham operated (lane e). The pancreatectomized rat has a blood glucose level of l70 mg/dl. A partial pancreatectomy (90%) also induces insulin deficiency, but in a milder form than the streptozotocin treatment. Adipsin mRNA is induced approximately 2-fold in the adipose tissue of these rats while the other mRNAs remain unchanged.

In another form of metabolic perturbation, rats were continuously infused for 48 hours with a glucose solution or with the vehicle alone. The results are shown in FIG. 2 where RNA was extracted from epidydimal fat pads. In the glucose model, rats were infused with dextrose (lane b) or saline (lane a) for 48 hours as described by Leahy et al., 77 J. Clin. Invest. 908 (1986). The dextrose-infused rats had blood glucose of 370 mg/dl compared to the control which had a glucose level of l46 mg/dl. The plasma insulin levels were l95 U/ml compared to 8 U/ml for the control. This glucose infusion protocol stimulates hyperglycemia, insulin release, and an insulin-induced anabolic state. The glucose infusion stimulated a l3-fold increase in GPD mRNA in adipose tissue. This observation is consistent with the function of GPD as a key regulatory step in lipogenesis. This increase is also attributed to the appearance of an mRNA slightly increased in size: approximately 2,700 bases instead of 3,500 bases. The structural basis for this larger GPD message is not known. Glucose infusion caused a 2-fold increase in the level of aP2 mRNA in adipose tissue, but led to a 65% decrease in the message for adipsin.

The results of these experiments are summarized in Table I below. The interpretation of these studies is facilitated by the fact that adipsin gene expression was examined in parallel to that of two other mRNAs which are expressed in a differentiation-dependent manner in adipocytes. The data clearly demonstrates that levels of these three mRNAs are regulated in response to fasting, refeeding, insulin deficient diabetes (caused by streptozotocin or partial pancreatectomy) or intravenous glucose infusion (which results in high circulating levels of both glucose and insulin). Importantly, despite their coordinate regulation during the process of adipocyte differentiation, these mRNAs are discordantly regulated in response to the above mentioned perturbations in vivo.

## Table 1.

### METABOLIC REGULATION OF FAT CELL mRNA'S IN THE RAT

| mRNA | CATABOLIC | | | ANABOLIC |
| --- | --- | --- | --- | --- |
| | Fasting | Streptozotocin | Pancreatectomy | Glucose Infusion |
| GPD | − | − | − | 12.8 X ↑ |
| ADN | 4.8 X ↑ | 3.9 X ↓ | 90 % ↓ | 65 % ↓ |
| AP2 | 40 % ↑ | − | − | 3.1 X ↓ |

Adipocyte GPD mRNA expression was largely unaltered with 3 days of fasting but increased markedly (l2-fold) after a continuous intravenous glucose infusion. These findings are consistent with the lipogenic function of this enzyme, parallel the available data for GPD enzyme activity, and demonstrate that the regulation of GPD activity in response to an anabolic stimulus is at least in part exerted at the mRNA level. Glucose infusion also induces aP2 expression, but somewhat less dramatically than GPD.

Adipsin mRNA is also modulated during many of these treatments, but the changes in expression differ greatly from those observed for GPD mRNA and aP2 mRNA. In fact, the data indicates that adipsin gene expression in mammals with normal metabolism correlates with the catabolic state. Specifically, it is induced by fasting and insulin deficient diabetes, and is inhibited by high levels of glucose and insulin. Thus, whatever the proximal signal responsible for this regulation, adipsin has a role in the catabolic state or the response to it.

The studies on adipsin gene expression in different models of obese rodents, in accordance with the present invention, demonstrate the utility of measuring the adipsin level. It has been found that adipsin mRNA and circulating adipsin protein are markedly suppressed in three different models of rodent obesity, two genetic and one acquired due to neonatal treatment with monosodium glutamate (MSG). In contrast, adipsin expression is unchanged in a model based on simple overfeeding.

The studies of the causes and consequences of obesity, in accordance with the present invention, have utilized genetically obese strains of rodents. The two best characterized strains of obese mice, ob/ob and db/db are homozygous recessive at genetic loci residing on chromosomes 6 and 4 respectively. Both strains are characterized by massive obesity that becomes apparent within two weeks after birth. In addition to obesity, these animals have hyperphagia, hyperglycemia, hyperinsulinemia, impaired thermogenesis, as well as a wide variety of other defects. At present, the nature of the primary genetic defects which leads to these multiple phenotypes is unknown. It is now found that adipsin mLRNA expresssion in adipose cells is massively reduced in both strains of obese mice representing one of the first examples of impaired gene expression in these animals, and by far the most severe biochemical defect described.

The expression of these genes in the fat tissue of animals was examined in two distinct genetic models of obesity and obesity-linked diabetes: the db/db mouse and the ob/ob mouse. FIG. 3 shows blotting analysis of epidydimal fat RNA from the db/db mouse and its congenic control. The congenic controls are genetically identical to the db/db and ob/ob mice except for the one gene which renders the animal obese. For GPD and aP2, the levels of mRNA are similar in the ad libidum fed states in control and db/db mice. Starvation caused a drop in GPD mRNA levels in both strains and both recover upon refeeding. Although the decrease in GPD mRNA is greater in the control animals (8-fold versus 4-fold), most of this difference can be ascribed to a generalized loss of mRNA during the fasting protocol. This is evident from the l3-fold fall in actin message in the control strain and a smaller (2-fold) fall in actin in the starved db/db animals. This general loss of mRNA per unit of total RNA, also confirmed by $^3$H-poly U hybridization and suggests that the decrease in GPD mRNA, as a fraction of total mRNA, was approximately 50% in both control and db/db animals. Enzyme specific activity measurements also display an almost 50% reduction in GPD activity during the fast in both control and obese animals. The response of GPD mRNA to fasting and feeding was similar in the control and obese mice.

A profound difference is observed in adipsin mRNA levels between the db/db mice and their congenic controls. In the fed states, this difference is at least l00-fold, although in some experiments the signal from the obese mice was too low to quantitate. Furthermore, starvation of the obese mice, which ought to reverse any changes obligatorily linked to the hyperphagia of these animals, fails to substantially increase the adipsin mRNA level. Starvation of the congenic control caused a 2-fold increase in adipsin mRNA, a somewhat smaller increase than was observed in the rat. This smaller increase is likely due to the greater general loss of mRNA during fasting in the mouse, as compared to the rat. To determine whether the very weak adipsin signals observed in the db/db strain represented an mRNA of similar size to that in the congenic control mouse, a lane containing the db/db mRNA was electrophoresed next to a lane containing l/50th as much mRNA from the control mouse as shown in FIG. 4. These were blotted and hybridized to probes of very high specific activity. It can be seen that these mRNAs appear to be the same size because of their adjacent positions on the gel.

It is important to know whether this very large change in adipsin mRNA is specific to the db/db model or whether it can be observed in other strains of obese mice. FIG. 5 shows the levels of these 3 fat cell mRNAs and actin mRNA in epidydimal fat RNA isolated from the ob/ob strain of obese mice. Lane e is a 50-fold shorter exposure of lane a to permit a more direct comparison of mRNA size in ob/ob and the control The arrows in FIG. 5 point to the adipsin species in the ob/ob mouse and the control mouse. A strain congenic to ob/ob animals is analyzed for comparison. It is evident that the mRNA levels are similar for GPD and aP2 in the two strains. However, a severe reduction is again observed in adipsin mRNA; the decrease is estimated to be at least l00-fold. Moreover, it is evident that in this case the residual adipsin mRNA in the ob/ob animals has a different size, l.25kb instead of l.lkb. Neither this difference in size nor quantity can be reversed by fasting the on/ob animals for 3 days.

Further, the severity of the defect in adipsin expression, and the fact that it is not reversed with 3 days of caloric restriction, indicate that this suppression is not obligatorily related to hyperphagia and its metabolic sequalae. This suggests that adipsin deficiency, rather than simply being a consequence of obesity or hyperphagia, plays a pathogenetically important role in the genesis of some of the features of these animals. In this regard, it is intriguing that the ob/ob mice express an adipsin mRNA which is altered in size compared to both its congenic normal strain and to other obese mice examined. The molecular basis for this difference has not been established.

The question of whether these differences in mRNA levels reflect the amounts of adipsin protein was examined. Adipsin protein can be measured by Western blotting using antisera prepared against specific peptides of the adipsin molecule. The antisera is directed against synthetic peptides corresponding to regions of adipsin deduced from the nucleotide sequence. The peptides used as antigens were selected by the following criteria. First, sequences unique to adipsin were chosen. Regions of adipsin showing extensive primary sequence homology with other members of the serine protease family were avoided to reduce the possibility of cross-reaction with other proteases. Secondly, peptides expected to lie on the surface of the protein and hence, be accessible to antibody molecules, were identified by aligning the sequence of adipsin to three-dimensional structures established for the other serine proteases. K. Cook et al., (l987); J. Greer, l53 J. Mol. Biol. l027 (l98l); R.M. Stroud et al., Cold Spring Harbor Symp. 36 Quant. Biol. l25 (l97l).

Using these sera, it has been shown that adipsin is secreted from cultured adipocytes and can be found both in adipose tissue and peripheral blood. In FIG. 6, ADN denotes the positions of the 37 and 44 kd forms of adipsin from adipocyte conditioned medium. Tissue extracts contain adipsin of 37-38 kd. FIG. 6 illustrates that adipsin protein is evident as a distinct band (38 kd) in adipose tissue extracts from both control strains congenic to the obese animals (lanes a-c and g). Both obese strains yield a sharper band at approximately 40 kd compared to the broader signal in the control strains (lanes d-f and h-j). Adipsin is virtually undetectable in extracts from the fat tissue of both obese strains. This difference between obese and congenic controls is estimated to be at least l00-fold in both the db/db and ob/ob mice. Moreover, the proteins appear to have a somewhat different pattern in gels when compared to the controls. Since the change in size between the 28 kd primary translation product and the protein observed secreted from cells is primarily due to glycosylation, the differences in adipsin mobility between the obese and normal animals may be due to altered (less heterogeneous) glycosylation. In summary, adipsin protein appears to be greatly altered quantitatively in these two genetic models of obesity.

To more closely examine the relationship between obesity and adipsin expression, two other animal models were examined. The MSG model was chosen to represent an acquired syndrome of rodent obesity. Neonatal mice injected subcutaneously with large amount of MSG develop a hypothalamic lesion that is rapidly followed by the development of obesity despite normal food intake. Recent studies suggest that impaired sympathetic nervous system activation of thermogenesis may contribute to the expanded adipose mass of these animals. Mice were studied l2 weeks after MSG treatment at which point epidydimal fat pad weight, which reflects overall obesity, was increased 3-fold over non-injected controls. Like the results obtained with the genetically obese animals, adipsin mRNA expression was reduced in the fat pads of MSG-treated animals. A decrease of approximately 20-fold relative to the levels in untreated animals was measured. Similarly, adipsin protein was markedly reduced in protein extracted from fat pads, as shown in FIG. 6, and in serum,, as shown in FIG. 7, from MSG-treated animals. No differences were observed in the electrophoretic mobilities of adipsin mRNA or protein in samples from this model as illustrated by FIGS. 6, 7 and 8.

This result demonstrates that adipsin deficiency can be seen in both genetic and acquired forms of obesity. It also raised the possibility that, whatever the molecular mechanism, adipsin gene expression is impaired in all models of obesity whenever the adipocyte is excessively enlarged. To probe this possibility, the "cafeteria-feeding" model of obesity was studied.

This second model of acquired obesity is representative of simple gluttony without an underlying neuroendocrine or metabolic defect. If the adipsin expression were found to be decreased in such a model, this might indicate that adipsin expression is reduced in any state in which adiposyte size is increased regardless of the underlying defect. To examine this, the "cafeteria-feeding" model was chosen. In this model, the animals are exposed to a frequently changed array of highly palatable foods such as cookies, potato chips, et. Under these conditions, they typically ingest more calories than when exposed to laboratory pellets. The resulting obesity may be viewed as being analagous to that arising through "simple gluttony" without an underlying metabolic defect. Normal 8-week-old Sprague Dawley (SD) rats that were "cafeteria-fed" for l2 weeks were studied. These animals weighed l0% more than chow fed controls, had plasma insulin levels 2-3 fold evelvated, and had epidydimal fat pads 2-3 times heavier than controls. Brown fat, the most important site of the adaptive thermogenesis that is known to occur in this model of overfeeding, was hypertrophied 2-fold over the control. Strikingly, and in contrast to our findings with the other obese animals, the abundance of adipsin mRNA in fat pads of these rats was not decreased. Adipsin mRNA actually increased 2-3 fold per unit total mRNA in the obese animals compared to the control, but was essentially identical when normalized to the actin signal (FIG.8). This finding appears to rule out a model in which the overfilled adipocyte always exhibits suppressed adipsin mRNA expresssion. Instead, it raised the intriguing possibility that the expression of adipsin mRNA and protein may distinguish the adipocyte that is enlarged due to "simple gluttony" from that which is enlarged due to an underlying metabolic defect.

All of the models of obesity utilized here are complex, with multiple endocrine derangement. However, the clearest general feature which distinguished the db/db, ob/ob and MSG models, on the one hand, from the cafeteria-fed animals on the other related to energy utilization and more specifically, to diet-induced thermogenesis. This physiologically important component of overall heat production and oxygen consumption in mammals appears to serve as a physiologic defense against excessive weight gain. Because obesity is fundamentally a disorder of energy balance, the status of diet-induced thermogenesis had been assessed in many models of obesity. It is well established that cafeteria-fed animals respond to overfeeding with increased sympathetic nervous system activity and, as a consequence, increased thermogenesis (i.e. heat production). This adaptive increase in diet-induced thermogenesis in the "cafeteria-fed" rat stands in direct contrast to observation in the three other models of obesity in which affected animals display defective thermogenesis compared to normal controls. Thus, whereas cafeteria-fed rats become obese despite an adaptive increase in energy expenditure, the obesity of the three other models is believed to be in large measure a result of impaired energy expenditure.

An analogous pattern of altered adipsin expression occurs in obese mammalian species, such as man. Thus, this molecule serves as a highly useful marker for characterizing obese states or distinguishing different classes of obese patients. The application of this technology to humans involves producing the antibodies to human adipsin in order to measure the level of adipsin in the patient's blood, and synthesizing the adipsin protein to be administered to the patient.

For determination of adipsin levels in the blood and adipose tissue of patients, the preferred method is radioimmunoassay, using human adipsin labelled with $^{125}$I by the chloramine T method and antibodies that interact with human adipsin that are raised in rabbits. Antibody-bound and free adipsin are separated by absorption to protein A-sepharose or by charcoal absorption. The adipsin level in serum is determined by comparing the displacement caused by the adipsin present in a patient's serum to that caused by known amounts of adipsin protein (i.e. a standard curve).

Other workable assays to determine adipsin levels employ nonradioactive ELISA assays using anti-adipsin antibodies and Western immuno-blots. In addition to human adipsin, assays exist which use mouse adipsin, adipsin isolated from other species, and adipsin fragments and analogues. Anti-adipsin antibodies which result when sheep and mice are immunized with human adipsin, mouse adipsin, adipsin from other species, and fragments and analogues of.the adipsin molecule will also be used to measure human adipsin levels.

The human adipsin cDNA probe is also used to determine restriction fragment length polymorphisms (RFLP's) that identify an individual whose propensity to gain weight stems from a genetic disorder. Since diabetes and obesity are closely linked disease states, adipsin RFLP's also predict an individual's propensity to develop diabetes. RFLP's are carried out by methods well known in the art. One example is as follows: RFLP is performed by removal of l0 ml of a patient's blood, isolation of DNA from leukocytes, digestion with restriction enzymes, electrophoresis of digested DNA, transfer to a filter, and Southern hybridization. Hybridizing fragments will be detected by autoradiography.

The antibodies to human adipsin used to detect and measure human adipsin protein are prepared in several ways. In the first method, peptides of l0-20 amino acids are synthesized corresponding to conserved regions of mouse adipsin. Cook et al. 82 Proc. Natl. Acad. Sci. USA 6480 (l985). These are conjugated to a carrier protein, keyhole limpet hemocyanin, and used to immunize rabbits by standard procedures. The antisera reacts with human adipsin in assays described below and may be used as whole sera or the immune antibodies are further purified on immobilized peptide columns by standard procedures.

In the second method, the entire mouse adipsin protein is overexpressed in a baculovirus expression vector system. The total mouse adipsin cDNA is linkered with BamHI linkers and inserted into Baculovirus vector pAc373 at the unique BamHI site. Summers and Smith, A Manuel of Methods for Baculovirus Vectors and Insect Cell Culture Procedures, U. of Texas College of Agriculture, l986. The expressed adipsin protein is purified by SDS-polyacrylamide gel electrophoresis and used to immunize rabbits by standard procedures. Whole sera or immunoglobulins immunoaffinity purified on mouse adipsin colummns are used for assay of human adipsin. Expression in <u>Spodoptera frugiperda</u> cells is as described in Summers and Smith, l986. Human adipsin cDNA is isolated and DNA sequence determined as discussed subsequently.

In the third method, peptides of l0-20 amino acids are synthesized corresponding to predicted external regions of human adipsin. These are conjugated to a carrier protein, keyhole limpet hemocyanin, and used to immunize rabbits by standard procedures. The antisera reacts with human adipsin in assays described below and may be used as whole sera or the immune antibodies may be further purified on immobilized peptide columns by standard procedures.

In the fourth method, the entire human adipsin protein is overexpressed in a baculovirus expression vector system. The total human adipsin cDNA is linkered with BamHI linkers and inserted into Baculovirus vector pAc373. Summers and Smith, l986. Expression in <u>S.frugiperda</u> cells is as described in Summers and Smith, l986. The protein is purified by SDS-polyacrylamide gel electrophoresis and used to immunize rabbits by standard procedures. Whole sera or immunoglobulins immunoaffinity purified on human adipsin columns are used for assay of human adipsin.

In order to isolate human adipsin cDNA, total RNA is prepared from fresh human adipose tissue and poly (A+) mRNA is isolated by oligo dT-cellulose chromatography. Then micrograms of this mRNA is used to prepare a cDNA library of approximately $3 \times 10^6$ placques in a lambda gt-l0 or gt-ll vector. Since mouse adipsin cDNA can detect the human adipsin gene by Southern blotting (see Figure 9), the mouse adipsin cDNA is used to screen the adipose tissue lambda gt-l0 library under condition of reduced hybridization and washing stringency. Hybridization is to $^{32}$P-labelled mouse adipsin cDNA and library placque lifts are done on nitrocellulose membranes. Conditions include, but are not limited to: hybridization at 42°C in 5xSSC (where SSC consist of 0.l5M NaCl, 0.l5M sodium citrate, and is at pH7.4), 0.l% SDS, 5xDenhardts (of which lx consists of 0.l% bovine serum albumen, 0.l% Ficoll 400 and 0.l% polyvinylpyrrolidone) and 100μg/ml denatured Salmon sperm DNA at 37°C for 48 hours. Washing is done at 0.lxSSC, 0.l%SDS at room temperature for l-3 hours, followed by autoradiography. Positive clones are placque purified and the DNA sequence is determined from one clone or a series of overlapping clones. The DNA sequence is determined by standard Sanger or Maxim-Gilbert techniques and yields directly the complete amino acid sequence of human adipsin protein. Human adipsin cDNA present in the lambda gt-II expression vector is detected by use of antibodies prepared against mouse adipsin. Briefly, antibodies prepared as described previously in methods I and 2 are reacted with replica lifts taken from the gt-II library. These are then reacted with $^{125}$I-labelled protein A, washed by standard conditions, and autoradiographed. Radioactive placque are then purified and the DNA sequence of these human adipsin clones are determined.

When adipsin deficiency is diagnosed in a patient by one of the above mentioned assays for adipsin in blood, adipsin is therapeutically administered to achieve restoration of blood levels to the normal range. The preferred dosage form of adipsin is human adipsin, produced biosynthetically in a mammalian cell expression system and purified using immunoaffinity columns with anti-adipsin antibodies. Other workable adipsin preparations are natural mouse adipsin, natural human adipsin, biosynthetic mouse adipsin, and natural variants of adipsin from other species. The preferred dosage range is 0.3 to I5 μg/kg of body weight given intravenously per day, but a workable therapeutic range is 0.I to 50 μg/kg body weight daily in recognition of varying degrees of adipsin deficiency and patient responsiveness to administered adipsin. The intravenous dosage form consists of adipsin in a physiologic saline solution at pH 7.4, and at a concentration designed to deliver the medically effective dose in 50 ml of solution over a 24-hour period. Alternate workable dosage forms include subcutaneous injection, intravasal administration by droplet or spray, and direct injection into fat depots to reduce the size of a local fat depot. Nasal administration will require 3-20 fold greater doses due to loss of bioavailability by the nasal route, direct injection requires smaller doses tailored to the size of the treated area, and subcutaneous administration requires the dose to be concentrated to a volume of less than I ml for convenient injection.

The preferred Method for synthesizing adipsin for therapeutic use involves expressing the fully glycosylated human adipsin protein in a cultured Chinese Hamster ovary (CHO) cell mammalian expression system. One workable method involves cloning the full length coding portion of the human adipsin cDNA into a pBR vector containing a metallothionine promoter and SV40 enhancer sequence as described by Greene et al., 23I Science II50 (I986). The ends of the adipsin cDNA insert are linkered with BamHI linkers before cloning into the vector. The expression vector containing adipsin cDNA is used to transform Chinese Hamster ovary cells (CHO-KI) by $CaPO_4$ precipitation. Cellular clones are screened for adipsin production and overproducing clones are selected, expanded and utilized for adipsin production in mass culture. Other workable mammalian cells in which these expression vectors are used include CV-I monkey kidney cells, and human Hela cells. Other workable non-mammalian cells in which the protein can be expressed through the use of suitable vectors are yeast, E. coli (Goeddel et al., 28I Nature 544 (I979)) and insect cells.

The invention may be embodied in other specific forms without departing from the essential characteristics thereof. The present embodiments are therefore to be considered in all respects as illustrative and not restrictive.

It is recognized that various modifications are possible when within the scope of the present invention as claimed.

**Claims**

1. An assay kit for adipsin levels for use in the diagnosis of genetic or metabolic abnormalities inducing obesity or diabetes.

2. An assay kit according to Claim 1 wherein the assay is an immunoassay.

3. An assay kit according to Claim 2 wherein the assay is a competition assay comprising human adipsin which has been labelled with $^{125}$I by the chloramine T method, rabbit anti-human-adipsin antibodies and a protein A-sepharose or charcoal separation means.

4. An assay kit according to Claim 2 or 3 comprising rabbit anti-human-adipsin antibodies raised with a conjugate of a carrier protein and a peptide of 10-20 amino acids corresponding to a conserved region of adipsin.

5. An assay kit according to Claim 4 wherein the carrier protein is keyhole limpet haemocyanin.

6. An assay kit according to Claim 2 or 3 comprising rabbit anti-human-adipsin antibodies raised with purified adipsin produced by the expression of adipsin cDNA in a baculovirus expression vector system.

EP 0 382 720 B1

7. An assay kit according to Claim 1 wherein the assay is a genetic assay comprising means to detect a restriction fragment length polymorphism (RFLP) or absence thereof in an adipsin-related nucleotide sequence.

8. An assay kit according to Claim 7 wherein the adipsin-related nucleotide sequence is genomic leukocyte DNA.

9. An assay kit according to Claim 1 wherein the assay is an ELISA.

10. A method of diagnosing genetic or metabolic disorders inducing obesity or diabetes, the method being carried out on a body sample separated from the body and comprising the detection of abnormally low levels of adipsin in the blood or adipose tissue or the detection of a restriction fragment length polymorphism (RFLP) in an adipsin-related nucleotide sequence.

11. Human adipsin for use in the treatment of genetic or metabolic disorders inducing obesity.


**Patentansprüche**

1. Testset für Adipsin-Spiegel zur Verwendung in der Diagnostik von genetischen oder metabolischen Anomalien, die zu Fettsucht oder Diabetes führen.

2. Testset gemäß Anspruch 1, bei dem der Test ein Immunoassay ist.

3. Testset gemäß Anspruch 2, bei dem der Test ein Vergleichstest ist, der menschliches Adipsin, welches mit $^{125}$I durch das Chloramin-T-Verfahren markiert war, Hasen-Antihuman-Adipsin-Antikörper und ein Protein-A-Sepharose oder Aktivkohle-Trennmittel enthält.

4. Testset gemäß Anspruch 2 oder 3, welches Hasen-Antihuman-Adipsin-Antikörper aufweist, die mit einer Verbindung aus einem Trägerprotein und einem Protein von 10 - 20 Aminosäuren, die einer erhaltenen Adipsinstelle entsprechen, gebildet wurden.

5. Testset gemäß Anspruch 4, bei dem das Trägerprotein Keyhole-limpet-Hämocyanin (KLH) ist.

6. Testset gemäß Anspruch 2 oder 3, bei dem die Hasen-Antihuman-Adipsin-Antikörper mit gereinigtem Adipsin gebildet sind, welches durch Expression von Adipsin-cDNA in einem Baculovirus-Expressions-Vector-System hergestellt wird.

7. Testset gemäß Anspruch 1, bei dem der Test ein genetischer Test ist, der Mittel umfaßt, um einen Polymorphismus der Länge von Restriktionsfragmenten (RFLP) oder dessen Abwesenheit in einer Adipsin-verwandten Nucleotid-Sequenz nachzuweisen.

8. Testset gemäß Anspruch 7, bei dem die Adipsin-verwandte Nucleotid-Sequenz genomische Leukozyten sind.

9. Testset gemäß Anspruch 1, bei dem der Test ein ELISA ist.

10. Verfahren zur Feststellung von genetischen oder metabolischen Störungen, die zu Fettsucht oder Diabetes führen, wobei das Verfahren an einer Körperprobe durchgeführt wird, die vom Körper getrennt wird, und wobei das Verfahren den Nachweis von anormal niedrigen Adipsin-Werten im Blut oder Adipose-Gewebe oder den Nachweis eines Polymorphismus der Länge von Restriktionsfragmenten (RFLP) in einer Adipsin-verwandten Nucleotid-Sequenz umfaßt.

11. Human-Adipsin zur Verwendung bei der Behandlung von genetischen oder metabolischen Störungen, die zu Fettsucht führen.


**Revendications**

1 - Nécessaire d'essai de taux d'adipsine utilisé dans le diagnostic d'anomalies génétiques ou métaboliques induisant une obésité ou du diabète.

2 - Nécessaire d'essai selon la revendication 1, dans lequel l'essai est un immunoessai.

3 - Nécessaire d'essai selon la revendication 2, dans lequel l'essai est un essai par compétition comprenant de l'adipsine humaine qui a été marquée avec du $^{125}$I par la méthode à la chloramine T, des anticorps de lapin anti-adipsine humaine et une protéine A-sépharose ou des moyens de séparation au charbon de bois.

4 - Nécessaire d'essai selon la revendication 2 ou 3, comprenant des anticorps de lapin anti-adipsine humaine dirigés avec un conjugué d'une protéine formant support et d'un peptide de 10 à 20 amino-acides correspondant à une région conservée de l'adipsine.

5 - Nécessaire d'essai selon la revendication 4, dans lequel la protéine support est une hémocyanine de patelle.

6 - Nécessaire d'essai selon la revendication 2 ou 3, comprenant des anticorps de lapin anti-adipsine humaine dirigés avec de l'adipsine purifiée produite par l'expression du cDNA de l'adipsine dans un système vecteur d'expression baculovirus.

7 - Nécessaire d'essai selon la revendication 1, dans lequel l'essai est un essai génétique comprenant des moyens de détection d'un polymorphisme en longueur des fragments de restriction (RFLP) ou d'une absence d'un tel polymorphisme dans un séquence de nucléotide apparentée à l'adipsine.

8 - Nécessaire d'essai selon la revendication 7, dans lequel la séquence de nucléotide apparentée à l'adipsine est un DNA de leutocyte génomique.

9 - Nécessaire d'essai selon la revendication 1, dans lequel l'essai est un ELISA.

10 - Procédé de diagnostic de désordres génétiques ou métaboliques induisant une obésité ou du diabète, ledit procédé étant mis en oeuvre sur un échantillon sanguin prélevé du corps et consistant en la détection de taux anormalement bas d'adipsine dans le sang ou dans le tissu adipeux ou dans la détection d'un polymorphisme en longueur des fragments de restriction (RFLP) dans une séquence de nucléotide apparentée à l'adipsine.

11 - Adipsine humaine pour l'utilisation dans le traitement de désordres génétiques ou métaboliques induisant une obésité.

FIG. 1

FIG. 2

FIG. 3

FIG. 4

FIG. 6

FIG. 7A

FIG. 7B

FIG. 7C

FIG. 8

EP 0 382 720 B1

FIG. 9

20